# EUROPEAN PATENT APPLICATION

(11) **EP 3 848 471 A1**
(43) Date of publication of application: **14.07.2021**
(21) Application number: 20151225.8
(22) Date of filing: 10.01.2020
(51) Int. Cl.: C12P 19/18, C12P 19/00, C12P 19/04

(54) **SEQUENTIAL FERMENTATIVE PRODUCTION OF OLIGOSACCHARIDES**

(71) Applicant: Chr. Hansen HMO GmbH, 53619 Rheinbreitbach (DE)
(72) Inventor: JENNEWEIN, Stefan, 53605 Bad Honnef (DE); PARSCHAT, Katja, 53173 Bonn (DE)

(57) **Abstract**

Disclosed is a process for the production of a desired oligosaccharide, the process comprises providing a genetically engineered microbial cell which possesses a saccharide importer for the uptake of an intermediate oligosaccharide, and an enzyme being able to convert the intermediated oligosaccharide by transferring a monosaccharide moiety from a donor substrate to the intermediate oligosaccharide; cultivating the genetically engineered microbial cell in the presence of an intermediate oligosaccharide to generate the desired oligosaccharide; and retrieving the desired oligosaccharide.

## Description

The present invention relates to processes for the production of saccharides. More specifically, the present invention relates to fermentative production of a desired oligosaccharide or desired polysaccharide, i.e. to the production of the desired oligosaccharide or the desired polysaccharide in a microbial cell.

### Background

The use of saccharides other than starch, cellulose and cane sugar gained a lot of interest lately. For example, certain oligosaccharides are used as functional food ingredients, nutritional additives, or as nutraceuticals. In particular prebiotic oligosaccharides became of high interest since they represent noncariogenic and nondigestible compounds stimulating the growth and development of human gastrointestinal microflora.

It has also long been known that human mother's milk, besides lactose, comprises a complex mixture of oligosaccharides called Human Milk Oligosaccharides (HMO). With respect to its oligosaccharide composition and the quantities of individual oligosaccharides, human breast milk is unique. Today, more than 150 structurally distinct human milk oligosaccharides have been characterized. The vast majority of human milk oligosaccharides is characterized by a lactose moiety at their reducing end. Many human milk oligosaccharides contain a fucose moiety and/or a sialic acid moiety at their non-reducing end. More generally speaking, the monosaccharides from which HMOs are derived are D-glucose, D-galactose, *N*-acetylglucosamine, L-fucose and sialic acid.

The most prominent human milk oligosaccharides are 2'-fucosyllactose (2'-FL) and 3-fucosyllactose (3-FL) which together can contribute to up to 1/3 of the total HMO fraction. Further prominent HMOs present in human milk are lacto-*N*-tetraose (LNT), lacto-*N*-*neo*tetraose (LNnT) and the lacto-*N*-fucopentaoses (LNFPs). Besides these neutral oligosaccharides that can be either fucosylated or non-fucosylated, also acidic HMOs comprising at least one sialic acid moiety can be found in human milk, such as for example 3'-sialyllactose (3'-SL), 6'-sialyllactose (6'-SL) and 3-fucosyl-3'-sialyllactose, disialyl-lacto-*N*-tetraose etc. The structures of these acidic HMOs are closely related to epitopes of epithelial cell surface glycoconjugates, the Lewis histoblood group antigens. The structural similarity of HMOs to epithelial epitopes accounts for the HMOs' protective properties against bacterial pathogens.

Besides above-mentioned local effects of HMOs in the intestinal tract, they have also been shown to elicit systemic effects in infants by entering the systemic circulation. In addition, the impact of HMOs on protein-carbohydrate interactions, e.g., selectin-leukocyte binding, can modulate immune responses and reduce inflammatory responses.

Due to their superior health benefits, the interest in HMOs as nutraceuticals has strongly increased over the past years. It is well established that HMOs contribute to the human body's defense mechanism against pathogens, to the establishment of a particular intestinal flora (microbiome), and to the postnatal stimulation of the immune system. Although HMOs are naturally consumed by infants, it is accepted that the beneficial effects of these oligosaccharides also occur if they are consumed in later life stages, i.e. during adolescence and thereafter.

Today, many oligosaccharides are synthesized *de novo* by *in vitro* biocatalytic glycosylation reactions using glycosyltransferases. Alternatively, oligosaccharides are obtained by chemical, physical and/or biological degradation of polysaccharides. For example, fructooligosaccharides (FOS) and galactooligosaccharides (GOS) are the most abundantly produced oligosaccharides. They are either synthesized or produced from plant polysaccharides.

Conventional chemical synthesis of oligosaccharides involves several reaction steps including elongation of an existing saccharide chain, purification of intermediate compounds, and - if applicable - further reaction steps such as - but not limited to - acetylating reactions and/or sulfating reactions. Such concept of a multi-step synthesis is unknown in the field microbial production of oligosaccharides.

The increasing demand of prebiotic oligosaccharides, in particular of HMOs, due to their beneficial properties renders a low-cost large-scale production of these saccharides highly desirable.

However, the main drawback today for wide-spread commercial use of many prebiotic and/or human milk oligosaccharides is the lack of effective oligosaccharide production. As mentioned above, oligosaccharides can be generated either by synthesis using biocatalysis or chemical engineering, or by polysaccharide depolymerization using physical, chemical or enzymatic methods.

Chemical synthesis of oligosaccharides has been proved to be challenging due to the presence of several hydroxyl groups of similar chemical reactivity in saccharide molecules. Thus, in chemical synthesis of oligosaccharides saccharide building blocks have first to be selectively protected in order to control the reactivity of the chemically similar groups, then coupled, and finally de-protected to obtain the desired oligosaccharide. Even if a small-scale synthesis of a desired oligosaccharide is possible, the thus developed synthetic routes for their synthesis are in general time-consuming, technically challenging and often prohibitively expensive. Thus, upscaling chemical oligosaccharide synthesis to a commercially viable industrial scale production is virtually impossible.

Enzymatic synthesis or the combination of chemical and enzymatic synthesis offer significant advantages over mere chemical synthesis routes. Several human milk oligosaccharides such as 2'-fucosyllactose, lacto-*N*-tetraose, lacto-*N*-*neo*tetraose or 3'-sialyllactose were already obtained by enzymatic synthesis. Yet, such chemical or biochemical synthesis of oligosaccharides is more difficult than the synthesis of other biopolymers such as peptides or nucleic acids. This renders biocatalysis or a combination of chemical and enzymatic synthesis of oligosaccharides nearly impossible to be implemented as an industrial scale production at reasonable costs. A major encounter that also contributes significantly to the cost in the large-scale production of oligosaccharides is the need to remove non-desired compounds such as non-desired oligosaccharides, donor substrate and acceptor substrates from the reaction mixture.

Besides biocatalysis, fermentative approaches for the production of oligosaccharides was successful, and several oligosaccharides - including HMOs - have been made available in considerable amounts by fermentative means.

In particular fermentative approaches for the production of HMOs are cost efficient in comparison to chemical or biochemical synthesis methods. Fermentative production of trisaccharide HMOs such as 2'-fucosyllactose and 3-fucosyllatose using metabolically engineered microorganisms are already established to provide these oligosaccharides in large volumes. However, even in fermentative processes by-products are synthesized due to the promiscuity of the enzymes employed in the microbial production of the desired oligosaccharide, chemical modifications of the carbohydrates and/or remaining starting materials which constitute contaminants in the final oligosaccharide product. These contaminants, also designated as by-products, are often structurally closely related to the desired oligosaccharide. Therefore, it is particularly difficult to separate the by-products from the desired oligosaccharide.

For example, the fermentative synthesis of lacto-N-neotetraose (LNnT) is a challenging process, because the non-reducing end of the molecule mimics the non-reducing end of lactose as both molecules possess a β-1,4-linked galactose residue at their non-reducing end. Notably, lactose is either the starting material or an intermediate in the fermentative production of LNnT. Unspecific reactions of glycosyltransferases employed y the microbial cell in the fermentative production of LNnT leads to an extensive formation of by-products such as pentaoses or hexaoses.

In addition, lacto-N-triose II (LNT-II) is an intermediate in the biosynthesis of LNnT. LNT-II can be efficiently exported from the LNnT-synthesizing microbial cell. Removal of LNT-II and other trisaccharide by-product, as well as the by-products formed upon the chain elongation of the LNnT product is challenging and expensive.

Another relevant problem is the formation of by-products when producing pentaoses such as the HMO lacto-*N*-fucopentaose III (LNFP-III) in a microbial cell. In a fermentative production process using single cells to synthesize LNT-II from lactose, LNnT from LNT-II and LNFP-III from LNnT, all by-products already occurring in the synthesis of LNnT can act as substrate for the final fucosylation reaction converting LNnT to LNFP-III. This leads to an intracellular mixture of fucosylated and non-fucosylated trioses, tetraoses, pentaoses other than LNFP-III, hexaoses and even larger oligosaccharides. These undesired by-products have to be removed from the preparation of the desired oligosaccharide such that the desired oligosaccharide can be provided in a substantially pure preparation, i.e. in a purity that meets requirements from regulatory authorities if the desired oligosaccharide is intended for human consumption. Preferably, a desired oligosaccharide has a purity of ≥ 80 %, ≥ 85 %, ≥ 90 %, ≥ 93 %, ≥ 95 %, ≥ 98 % or even ≥ 99 % in the substantially pure preparation of said desired oligosaccharide.

Oligosaccharides such as HMOs can be produced by fermentation using genetically engineered microbial cells and adding lactose to the fermentation medium as initial substrate for the enzymatic synthesis of the desired oligosaccharide. Oligosaccharides with up to six monosaccharides moieties can be produced in such a fermentation process. However, due to promiscuity of the glycosyltransferases used or by chemical modification of the carbohydrates or due to incomplete transformation of an intermediate oligosaccharide the final fermentation broth mostly contains a bunch of oligosaccharides of different length and composition. To gain a pure product with respect of the carbohydrates addition of specific hydrolases can solve some of the problems, but it is limited by the availability of specific enzymes. In some cases, it is even impossible to find enzymes specifically degrading a by-product but not the desired oligosaccharide because of structural similarities, e.g. in lactose and LNnT is the beta-1,4-glycosidic bond subject to hydrolysis by a beta-galactosidase in both carbohydrates.

Hence, there exists a great need for cost-efficient industrial scale processes to produce a desired oligosaccharide, preferably a prebiotic oligosaccharide, in particular a desired HMO.

The problem is solved by providing fermentative production processes for oligosaccharides, wherein the synthesis of the desired oligosaccharide does not occur from lactose as starting material in a single microbial cell but from an intermediate oligosaccharide which consists of at least three monosaccharide moieties. Such fermentative production process enables splitting of the individual biosynthetic steps for the formation of the desired oligosaccharide into individual fermentation steps utilizing different microbial cells for the synthesis of intermediate oligosaccharides and the desired oligosaccharides. The separate fermentation steps may be combined with process steps of recovering and/or purifying the intermediate oligosaccharide(s) before said intermediate oligosaccharide(s) is/are provided to the subsequent fermentation step. Using this approach, it is possible to avoid or at least reduce the presence of undesired saccharide by-products originating from unwanted glycosylation of intermediate oligosaccharides at the end of the fermentation process.

### Summary

Provided is a process for the production of a desired oligosaccharide comprising more than three monosaccharide moieties, wherein the process comprises providing a genetically engineered microbial cell that possesses (i) a saccharide importer which facilitates uptake of an oligosaccharide consisting of at least three monosaccharide moieties by the genetically engineered microbial cell, and (ii) an enzyme which is able to transfer a monosaccharide moiety form a donor substrate to the oligosaccharide consisting of at least three monosaccharide moieties; cultivating the genetically engineered microbial cell in a culture medium which contains the oligosaccharide consisting of at least three monosaccharide moieties for the genetically engineered microbial cell to take up the oligosaccharide consisting of at least three monosaccharide moieties and to transfer at least one monosaccharide moiety to the oligosaccharide consisting of at least three monosaccharide moieties, thereby synthesizing the desired saccharide comprising more than three monosaccharide moieties; and retrieving the desired saccharide from the culture medium and/or the genetically engineered microbial cell.

More specifically, the process may comprise production of larger oligosaccharides, i.e. of oligosaccharides consisting of at least four monosaccharide moieties, in a stepwise or sequential manner using at least two different genetically engineered microbial cells that are cultivated in separate compartments, wherein an oligosaccharide produced by one of the at least two different genetically engineered microbial cells serves as an educt for the production of another oligosaccharide, e.g. the desired oligosaccharide or another intermediate oligosaccharide, by the other of the at least two genetically engineered microbial cells.

When the process is performed in a sequential manner, a fermentation step builds on the product of a previous fermentation step. An oligosaccharide product - optionally containing undesired oligosaccharide by-products - is generated in an initial fermentation step. In a subsequent fermentation step, the oligosaccharide product of the initial fermentation step is further processed, either in that the undesired oligosaccharide by-products are degraded and/or in that the oligosaccharide of the product is used as an educt. At the end of the sequential fermentation a desired oligosaccharide is obtained at higher purity, i.e. accompanied by less undesired by-products, than by a process performing the production in a single compartment with different microorganism, or by using an individual microbial strain performing all enzymatic reactions required to obtain the desired oligosaccharide.

The sequential fermentation process utilizes specific biosynthetic compartments (comprising synthesis and/or specific degradation reaction) by employing different genetically engineered microbial cells (bacterial and/or eukaryotic microorganism (e.g. yeast) with suitable oligosaccharide up-take and/or export properties for desired oligosaccharides, intermediate oligosaccharides, oligosaccharide by-products and/or other undesired microbial products or carbohydrates.

### Brief description of the drawings

- FIG. 1: shows a schematic representation of an embodiment illustrating production of a desired oligosaccharide by sequential fermentation.
- FIG. 2: shows a schematic representation of an embodiment illustrating production of a desired oligosaccharide by sequential fermentation.
- FIG. 3: shows a schematic representation of an embodiment illustrating production of a desired oligosaccharide by sequential fermentation.
- FIG. 4: displays a schematic representation of an embodiment illustrating production of a desired oligosaccharide by sequential fermentation and its subsequent recovery.
- FIG. 5: illustrates the comparison of MRM spectra of different LNFP-III samples.
- FIG. 6: illustrated the comparison of MRM spectra of different LNnT samples.
- FIG. 7: illustrates the comparison of MRM spectra of different LNT-II samples.
- FIG. 8: illustrates the comparison of MRM spectra of different LNFP-II samples.

### Detailed description

The present invention provides a process for the production of a desired oligosaccharide. As used herein, the term "oligosaccharide" typically refers to a polymeric saccharide molecule consisting of at least three monosaccharide moieties, but no more than 12, preferably no more than 10 monosaccharide moieties, that are linked to one another by a glycosidic bond. The polymeric saccharide molecule may be a linear chain of monosaccharide moieties, or may be a branched molecule, wherein at least one monosaccharide moiety has at least three monosaccharide moieties bound to it by glycosidic bonds. The monosaccharide moieties can be selected from the group of aldoses (e.g. arabinose, xylose, ribose, desoxyribose, lyxose, glucose, idose, galactose, talose, allose, altrose, mannose, etc.), ketoses (e.g. ribulose, xylulose, fructose, sorbose, tagatose, etc.), deoxysugars (e.g. rhamnose, fucose, quinovose, etc.), deoxy-aminosugars (e.g. N-acetylglucosamine, N-acetyl-mannosamine, N-acetyl-galactosamine ect.), uronic acids (e.g. galacturonsäure, glucuronsäure ect.) and ketoaldonic acids (e.g. N-acetylneuraminic acid).

As used herein, the term "desired oligosaccharide" refers to the oligosaccharide that is intended to be produced. As used herein, the term "intermediate oligosaccharide" refers to an oligosaccharide that is used and/or synthesized for or during the process, but which is not the oligosaccharide constituting the final product to be produced by the process. It is understood that a given oligosaccharide may be the desired oligosaccharide in a process according to a particular embodiment, and that said given oligosaccharide may be an intermediate oligosaccharide in a process accordign to another particular embodiment.

The process comprises cultivating a genetically engineered microbial cell which is able to synthesize the desired oligosaccharide in a culture medium that contains an oligosaccharide that consists of at least three monosaccharide moieties, but which consists of less monosaccharide moieties than the desired oligosaccharide. Said oligosaccharide containing at least three monosaccharide moieties is designated as "intermediate oligosaccharide". Preferably, said intermediate oligosaccharide consists of one or two monosaccharide moieties less than the desired oligosaccharide, because in the process for producing the desired oligosaccharide, one, two or emore monosaccharide moieties are linked to the intermediate oligosaccharide to becom the desired oligosaccharide.

Table 1 provides a non-limiting list of desired oligosaccharides and corresponding intermediate oligosaccharides that can be produced by the process of the present invention.

**Table 1: List of desired oligosaccharides that can be produced by using the process of the invention, and the intermediate oligosaccharides to be provided to the culture medium.**

| Intermediate oligosaccharide | Desired oligosaccharide | Structure of desired oligosaccharide |
|---|---|---|
| 2'-Fucosyllactose | 2',3-Difucosyllactose | Fuc(α1-2)Gal(β1-4)Glu ; Fuc(α1-3) |
| Lacto-N-triose II | Lacto-*N*-tetraose | Gal(β1-3)GlcNAc(β1-3)Gal(β1-4)Glu |
| Lacto-N-triose II | Lacto-*N-neo*tetraose | Gal(β1-4)GlcNAc(β1-3)Gal(β1-4)Glu |
| Lacto-*N*-tetraose | Lacto-*N-*fucopentaose I | Fuc(α1-2)Gal(β1-3)GlcNAc(β1-3)Gal(β1-4)Glu |
| Lacto-*N*-tetraose | HBGA typ A1 | GalNac(α1-3)Gal(β1-3)GlcNAc(β1-3)Gal(β1-4)Glu ; Fuc(α1-2) |
| Lacto-N-fucopentaose I | HBGA typ A1 | GalNac(α1-3)Gal(β1-3)GlcNAc(β1-3)Gal(β1-4)Glu ; Fuc(α1-2) |
| Lacto-*N*-tetraose | HBGA typ B1 | Gal (α1-3)Gal(β1-3)GlcNAc(β1-3)Gal(β1-4)Glu ; Fuc(α1-2) |
| Lacto-N-fucopentaose I | HBGA typ B1 | Gal (α1-3)Gal(β1-3)GlcNAc(β1-3)Gal(β1-4)Glu ; Fuc(α1-2) |
| Lacto-*N-neo*tetraose | HBGA typ A2 | GalNac(α1-3)Gal(β1-4)GlcNAc(β1-3)Gal(β1-4)Glu ; Fuc(α1-2) |
| Lacto-N-neofucpentaose I | HBGA typ A2 | GalNac(α1-3)Gal(β1-4)GlcNAc(β1-3)Gal(β1-4)Glu ; Fuc(α1-2) |
| Lacto-*N-neo*tetraose | HBGA typ B2 | Gal(α1-3)Gal(β1-4)GlcNAc(β1-3)Gal(β1-4)Glu ; Fuc(α1-2) |
| Lacto-N-neofucpentaose I | HBGA typ B2 | Gal(α1-3)Gal(β1-4)GlcNAc(β1-3)Gal(β1-4)Glu ; Fuc(α1-2) |
| Lacto-*N-neo*tetraose | Lacto-*N-neo*fucopentaose I | Fuc(α1-2)Gal(β1-4)GlcNAc(β1-3)Gal(β1-4)Glu |
| Lacto-*N*-tetraose | Lacto-*N-*fucopentaose II | Gal(β1-3)GlcNAc(β1-3)Gal(β1-4)Glu ; Fuc(α1-4) |
| Lacto-*N-neo*tetraose | Lacto-*N-*fucopentaose III | Gal(β1-4)GlcNAc(β1-3)Gal(β1-4)Glu ; Fuc(α1-3) |
| Lacto-*N*-tetraose | Lacto-*N-*fucopentaose V | Gal(β1-3)GlcNAc(β1-3)Gal(β1-4)Glu ; Fuc(α1-3) |
| Lacto-*N*-tetraose | Lacto-*N-*difucohexaose I | Fuc(α1-2)Gal(β1-3)GlcNAc(β1-3)Gal(β1-4)Glu ; Fuc(α1-4) |
| Lacto-N-fucopentaose I | Lacto-N-difucohexaose I | Fuc(α1-2)Gal(β1-3)GlcNAc(β1-3)Gal(β1-4)Glu ; Fuc(α1-4) |
| Lacto-*N*-tetraose | Lacto-*N-*difucohexaose II | Gal(β1-3)GlcNAc(β1-3)Gal(β1-4)Glu ;                                    ; Fuc(α1-4)                        Fuc(α1-3) |
| Lacto-N-fucopentaose V | Lacto-N-difucohexaose II | Gal(β1-3)GlcNAc(β1-3)Gal(β1-4)Glu ; ; Fuc(α1-4) Fuc(α1-3) |
| Lacto-*N-neo*tetraose | Lacto-*N-*difucohexaose III | Gal(β1-4)GlcNAc(β1-3)Gal(β1-4)Glu ; ; Fuc(α1-3) Fuc(α1-3) |
| Lacto-*N*-tetraose | Lacto-*N*-hexaose | Gal(β 1-4)GlcNAc(β1-6)Gal(β1-4)Glu ; Gal(β1-3)GlcNAc(β1-3) |
| Lacto-*N-neo*tetraose | Lacto-*N*-neohexaose | Gal(β1-4)GlcNAc(β1-6)Gal(β1-4)Glu ; Gal(β1-4)GlcNAc(β1-3) |
| Lacto-*N-neo*tetraose | *para*-Lacto-*N-*hexaose | Gal(β1-3)GlcNAc(β1-3)Gal(β1-4)GlcNAc(β1-3)Gal(β1-4)Glu |
| Lacto-*N-neo*tetraose | *para-*Lacto-*N-neo*hexaose | Gal(β1-4)GlcNAc(β1-3)Gal(β1-4)GlcNAc(β1-3)Gal(β1-4)Glu |
| Lacto-*N*-tetraose | Lacto-*N-*sialylpentaose a | Neu5Ac(α2-3)Gal(β1-3)GlcNAc(β1-3)Gal(β1-4)Glu |
| Lacto-*N*-tetraose | Lacto-*N-*sialylpentaose b | Gal(β1-3)GlcNAc(β1-3)Gal(β1-4)Glu ; |
| | | Neu5Ac(α2-6) |
| Lacto-*N-neo*tetraose | Lacto-*N-*sialylpentaose c | Neu5Ac(α2-6)Gal(β1-4)GlcNAc(β1-3)Gal(β1-4)Glu |
| Lacto-*N*-tetraose | Fucosyl-lacto-*N-*sialylpentaose a | Neu5Ac(α2-3)Gal(β1-3)GlcNAc(β1-3)Gal(β1-4)Glu ; Fuc(α1-4) |
| Lacto-*N*-tetraose | Fucosyl-lacto-*N-*sialylpentaose b | Fuc(α1-2)Gal(β1-3)GlcNAc(β1-3)Gal(β1-4)Glu ; Neu5Ac(α2-6) |
| Lacto-*N*-tetraose | Fucosyl-lacto-*N-*sialylpentaose c | Neu5Ac(α2-3)Gal(β1-3)GlcNAc(β1-3)Gal(β1-4)Glu ; Fuc(α1-3) |
| Lacto-*N-neo*tetraose | Disialyl-lacto-*N-*tetraose | Neu5Ac(α2-3)Gal(β1-4)GlcNAc(β1-3)Gal(β1-4)Glu ; Neu5Ac(α2-6) |
| Lacto-*N-neo*tetraose | Disialyl-lacto-*N-*fucopentaose | Neu5Ac(α2-3)Gal(β1-4)GlcNAc(β1-3)Gal(β1-4)Glu ; ; Neu5Ac(α2-6) Fuc(α1-3) |
| 3-Fucosyllactose | 3-Fucosyl-3'-sialyllactose | Neu5Ac(α2-3)Gal(β1-4)Glu ; Fuc(α1-3) |
| 3-Fucosyllactose | 3-Fucosyl-6'-sialyllactose | Neu5Ac(α2-6)Gal(β 1-4)Glu ; Fuc(α1-3) |

The process comprises providing a genetically engineered microbial cell that possesses a sacchride importer for the uptake of an intermediate oligosaccharide consisting of at least three monosaccharide moieties. The intermediate oligosacchride is obtained from an exogenous source. Hence, the intermediate oligosacchride is provided to the culture medium and internalized or taken up be the genetically engineered microbial cell. Uptake of the intermediate oligosacchride be the microbial cell may occur by diffusion across the cell membrane.

An important aspect of the process disclosed herein is an effective transport of the intermediate oligosaccharide in and/or out of the microbial cells utilize din the process, as well as an effective transsport of the desired oligosacchride out of the microbial cell for its synthesis. This uptake (import) and export mechanisms may either occur naturally in the microbial cells or may have been introduced into the microbial cells by means of genetic engineering leading to the expression of a suitable importer and/or exporter.

In specific embodiments, the internalization of the intermediate oligosacchride into the genetically engineered microbial cell for the production of the desired oligosaccharide is facilitated in that a sacchride import system for the import system for the intermediate oligosaccharide is implemented in the genetically engineered microbial cell. Hence, the genetically engineered microbial cell possess a saccharide importer for the uptake of the intermediated oligosacchride.

For genetic engineering of a microbial cell to possess a saccharide importer, genes encoding saccharide importers for comples oligosaccharides sucha s HMOs can be obtained from the genome of bacteria which degrade such oligosaccharides intracellularly. Such bacteria can be found e.g. as symbionts in the colon or the rumen of mammalians but also in insect guts. Also, transport proteins effectively facilitating the uptake of complex oligosaccharides with at least three monosaccharide moieties such as lacto-N-triose, lacto-N-tetraose or lacto-N-neotetraose can be found in bacteria colonizing the colon of breast-fed infants.

In a preferred embodiment, the internalization of the extracellular intermediate oligosaccharide is facilitated by a saccharide importer that is heterologous to the microbial cell. Genes or functional DNA sequences encoding proteins that form a saccharide import system are expressed in the genetically engineered microbial cell. To this end, the nucleotide sequences encoding and expressing said saccharide importer can be integrated into the genome of the microbial cell or they can be transcribed from an extrachromosomal and/or episomal vector.

Several gut colonizing bacteria e.g. *Bifidobacteria* and *Lactobacilli* are known to be able grow on human milk oligosaccharides as sole carbon source. Degradation of complex human milk oligosaccharides can be achieved in two ways. Bacteria can secrete glycosidases in the medium to hydrolyze the human milk oligosaccharides into mono- and disaccharides which then can be imported by well-known and established carbohydrate uptake systems. However, several bifidobacterial species are known to internalize complex oligosaccharides (Garrido, D. et al. (2015). Comparative transcriptomics reveals key differences in the response to milk oligosaccharides of infant gut-associated bifidobacteria. Scientific reports, 5, 13517); (Özcan, E., & Sela, D. A. (2018). Inefficient metabolism of the human milk oligosaccharides lacto-N-tetraose and lacto-N-neotetraose shifts Bifidobacterium longum subsp. infantis physiology. Frontiers in Nutrition, 5, 46.); (James, K. et al. (2016). Bifidobacterium breve UCC2003 metabolises the human milk oligosaccharides lacto-N-tetraose and lacto-*N*-*neo*tetraose through overlapping, yet distinct pathways. *Scientific reports, 6,* 38560). Transport systems used by these bacteria comprise an ATP dependent permease with 2-3 auxiliary membrane / ATPase subunits and an associated extracytoplasmatic solute binding protein. The solute binding proteins were shown to be highly promiscuous in binding carbohydrate chains accepting oligosaccharides with two to eight monosaccharide units (Garrido, D. et al. (2011). Oligosaccharide binding proteins from Bifidobacterium longum subsp. infantis reveal a preference for host glycans. PLoS One, 6(3), e17315.).

An *in vivo* import system has been established in a *B. longum* strain expressing cytoplasmatic hydrolases to cleave human milk oligosaccharides intracellularly. Expression of heterologous genes encoding oligosaccharide transporters enables the strain to grow on specific human milk oligosaccharides (Sakanaka, M. et al. (2019). Evolutionary adaptation in fucosyllactose uptake systems supports bifidobacteria-infant symbiosis. Science advances, 5(8), eaaw7696). A fructose ABC-importer from the gram-positive bacterium *B. longum* could complement a fructose-uptake deficient *E. coli* strain (Wei, X. et al. (2012). Fructose uptake in Bifidobacterium longum NCC2705 is mediated by an ATP-binding cassette transporter. Journal of Biological Chemistry, 287(1), 357-367). However, HMO import systems from Bifidobacteria or other bacteria known to metabolize HMOs have not been cloned in a functional manner into *E. coli* until now. Genes encoding proteins resembling transport systems for HMO uptake are described for species of *Bifidobacterium longum* and *B*. *breve.*

When attempting to define the composition of the human gut microbiota it has to be stated that a high amount of bacterial species living in the human gut are uncultivable, thus the entire repertoire of metabolic traits including import systems on HMOs is undefined today (Almeida, A. et al. (2019). A new genomic blueprint of the human gut microbiota. Nature, 568(7753), 499). By searching the metagenome of stools of breast-fed infants the possibility to find HMO import facilitating proteins is not unimportant. By identifying and functional expression of a not yet described import system in the cell to produce the desired oligosaccharide the problem of the internalization of the intermediate oligosaccharide can be solved.

The genes or nucleotide sequences encoding saccharide importers for oligosaccharides with at least three monosaccharide units can be obtained from metagenomes that were isolated from appropriate environments or from bacterial strains known to harbor such oligosaccharide up-take mechanisms or combinations thereof.

Gram-negative bacteria, such as *E. coli,* have a bi-layer cell membrane and ABC transporters connect the cytoplasm with the periplasm. Up-take of complex oligosaccharides into the periplasm through the outer membrane are facilitated by porins. Porins are proteins that form a channel allowing the diffusion of large or charged molecules across the outer membrane. An example of such an oligosaccharide porin is ChiP in *E. coli* a chitooligosaccharide specific outer-membrane porin for (GlcNAc)₃₋₆. (Verma, S. C., & Mahadevan, S. (2012). The chbG gene of the chito-biose (chb) operon of Escherichia coli encodes a chitooligosaccharide deacetylase. Journal of Bacteriology, 194(18), 4959-4971). Another example for a porin is RafY from *E. coli,* shown to form a carbohydrate unspecific large pore facilitation the diffusion of oligosaccharides up to hexoses over the outer E. coli membrane (Andersen, C. et al. (1998). The porin RafY encoded by the raffinose plasmid pRSD2 of Escherichia coli forms a general diffusion pore and not a carbohydrate-specific porin. European Journal of Biochemistry, 254(3), 679-684).

In addition to a saccharide importer for up-take of the intermediate oligosaccharide into the cytoplasm across the inner membrane, a pore forming protein facilitating the transport of the intermediate oligosaccharide across the outer membrane of a microbial cell such as *E. coli* can be implemented to improve uptake of the intermediate oligosaccharide by the microbial cell.

In an embodiment, the microbial cell possesses a functional gene - or an equivalent nucleotide sequence - encoding and expressing or overexpressing the pore forming protein. The functional gene may be endogenous to the microbial cell or the protein-coding region of said gene may originate from a different species.

The process comprises the synthesis of the desired oligosaccharide by a genetically engineered mcirobial cell that is provided. Said genetically engineered microbial cell comprises an enzyme which is able to transfer a monosaccharide moiety from a donor substrate to the intermediate oligosaccharide. In certain embodiments, the genetically engineered microbial is a cell that has been genetically engineered to possess an enzyme which is able to transfer a monosaccharide moiety from a donor substrate to the intermediate oligosaccharide in that said genetically engineered microbial cell comprises a functional gene for the expression of an enzyme that is able to transfer a monosaccharide from a donor substrate to the intermediate oligosaccharide.

The functional gene for the expression of an enzyme that is able to transfer a monosaccharide moiety from a donor substrate to the intermediate oligosaccharide is a nucleic acid sequence that encodes the amino acid sequence(s) of said enzyme. The functional nucleic acid sequence further comprises expression control sequences. Said expression control sequences are opearbly linked to said nucleic acid sequence(s) that encodes the amino acid sequence(s) of said enzyme, and mediate expression of the nucleic acid sequence(s) that encodes the amino acid sequence(s) of said enzyme. The expression control sequences may be selected from the group consisting of promoters, enhancers and terminators.

The functional genes or equivalent DNA sequences which confer the enzymatic capability to add a monosaccharide moiety to an internalized intermediate oligosaccharide to the genetically modified cell can be a homologous nucleotide acid sequence or a heterologous nucleotide sequences originating from plants, animals, bacteria, archaea, fungi or viruses.

The enzyme that is able to transfer a monosaccharide moiety from a donor substrate to the intermediate oligosaccharide may be a glycosyltransferase or a transglycosidase. Examples of glycosyltransferases and transglycosidases are described in the Carbohydrate Active Enzymes database (CAZy) (http://www.cazy.org) and in literature, but sequences of natural occurring or engineered glycosyltransferases and transglycosidases are not limited to these examples.

The glycosyltransferase catalyzes the transfer of a monosaccharide moiety from a nucleotide-activated sugar as donor substrate to an acceptor molecule, e.g. an intermediate oligosaccharide. The glycosyltransferase may be selected from the group of glycosyltransferases consisting of galactosyltransferases, glucosaminyl-transferases, sialyltransferases, *N*-acetylglucosaminyltransferases, N-acetylgalacto-saminyltransferases, glucuronosyltransferases, mannosyltransferases, xylosyl-transferases and fucosyltransferases. In specific embodiments, the glycosyltransferase is a α-1,2-mannosyltransferase, β-1,4-xylosyltransferase, β-1,3-*N*-acetyl-glucosaminyltransferase, β-1,6-*N*-acetylglucosaminyltransferase, β-1,3-*N*-acetyl-galactosaminyltransferase, β-1,4-*N*-acetylgalactosaminyltransferase, α-1,3-*N-*acetylgalactosaminyltransferase, β-1,3-galactosyltransferases, β-1,4-galactosyltransferase, β-1,6-galactosyltransferase, α-1,3-glucosyltransferase, α-4-glucosyl-transferase, α-2,3-sialyltransferase, α-2,6-sialyltansferase, α-2,8-sialyltansferase, α-1,2-fucosyltransferase, α-1,3-fucosyltransferase or α-1,3/1,4-fucosyltransferase.

The donor substrate for a transfer of a monosaccharide moiety may be a nucleotide activated sugar or a saccharide, preferably an oligosaccharide. Nucleotide activated sugars are donor substrates for monosaccharide moieties that are used by glycosyltransferases. The nucleotide activated sugar may be GDP-fucose, UDP-galactose, UDP-glucose, CMP-N-acetylneuraminic acid, GDP-mannose, UDP-N-acetylgalactosamine or UDP-N-acetylglucosamine. It is understood that a fucosyltransferase utilizes GDP-fucose as donor substrate for the transfer of a fucose moietyl from GDP-fucose to an acceptor molecule, whereas a galactosyltransferase utilizes UDP-galactose as donor substrate for the transfer of a galactose moiety to an acceptor substrate, etc.

The nucleotide activated sugars that are used by glycosyltransferases as donor substrate are synthesized by the genetically engineered microbial cell in a *de novo* biosynthesis pathway and/or by using a salvage pathway.

When the genetically engineered mcirobial cell synthesizes the nucleotide activated sugars *de novo,* the microbial cell possesses the enzymes constituing the metabolic pathway for synthesizing the nucleotide activated sugar de novo from a simple carbon source such as glucose, sucrose, fructose, glycerol or the like.

In certain embodiment, the genetically engineered mcirobial cell that synthesizes the nucleotide activated sugars *de novo* has been genetically engineered to contain and express the genes that encode all enzymes for the *de novo* biosynthesis of the nucleotide activated sugar. Said genes may be endogenous to the microbial cell or any one of said genes can originate from other organisms and being introduced into the microbial cell to be transcribed and translated in a functional manner.

The monosaccharides that are used as substrates for the formation of nucleotide activated sugars in a salvage pathway can be taken up by the micobial cell passively or the internalization of the monosaccharide can be facilitated by transporter, preferbly by overexpression of endogenous genes encoding such transporter proteins and/or by expression of a heterologus gene encoding such transprot protein. Examples for monosaccharide transporters for uptake of a monosaccharide by a microbial cell are the fucose permease FucP or the sialic acid transporter NanT from *E. coli.*

When using a salvage pathway for biosynthesis of the nucleotide activated sugar as donor substrate, the microbial cell comprises enzymes which catalyze the transfer of a nucleotide to a monosaccharide. Examples for such enzymes are the bifunctional fucokinase/L-fucose-1-P-guanylyltransferase (EC 2.7.7.30) catalyzing a kinase and a pyrophosphatase reaction to form GDP-fucose e.g. FKP from *Bacteroides fragilis* (WO 2010/070 104 A1) and the N-acetylneuraminate cytidyltransferase (EC 2.7.7.43) forming CMP-neuraminic acid.

Said genetically engineered microbial cell comprises a functional nucleic acid sequence for the a transporter which facilitates uptake of the intermediate oligosaccharide consisting of at least three monosaccharide moieties but less monosaccharide moieties than the desired oligosaccharide.

In another embodiment the genetically engineered microbial cell contains a transglycosidase. The transglycosidase may be a heterologous transglycosidase. Hence, the genetically engineered microbial cell may possess a functional gene encoding a transglycosidase or an equivalent nucleotide sequence to said functional gene. Transglycosidases are enzymes which are able to catalyze the hydrolysis of disaccharides or oligosaccharides (glycosidase activity) and in a reverse reaction transfers a glycosidic residue from a glycoside donor to an acceptor substrate.

Glycosidases with transglycosidase activity can either be exo-acting or endo-acting on the carbohydrate substrate. Examples for glycosidases with transglucosidase activity are e.g. described for cellobiases, fucosidases, sialidases, glucosidases, galactosidases, lacto-*N*-biosidases, and chitinases.

In a specific embodiment, a transglycosidase for the biosynthesis of human milk oligosaccharides can be selected from the group consisting of β-1,3-galactosidases, β-1,4-galactosidases, β-1,6-galactosidases, α-1,2-fucosidases, α-1,3-fucosyidases, α-2,3-sialidases, α-2,6-sialidases, and β-N-acetylhexosaminidases.

Nucleotide sequences encoding a transglycosidase enzyme for providing a genetically engineered microbial cell for the production of a desired oligosaccharide can either be found in nature or it can be modified to favor the transglycosylation reaction against the glycosidase reaction using genetic techniques. A person of ordinary skilled in the art knows how to modify the nucleotide sequence to obtain an enzyme with enhanced transglycosidase activity (Zeuner, B. et al. (2019). Synthesis of human milk oligosaccharides: Protein engineering strategies for improved enzymatic transglycosylation. Molecules, 24(11), 2033).

Preferably, the enzyme used for transglycosylation in the microbial cell should express no or only very low hydrolase activity towards the acceptor substrate. The hydrolase activity should be less than 10%, 9%, 8%, 7%, 6%, 5%, 4%, 3%, 2%, 1%, 0.5%, 0.1%, preferably less than 0.01%, compared to its transglycosidase activity.

In some embodiments the transglycosidase used is a α-1,3/4-fucosidase catalyzing the transfer of a L-fucose moiety from a donor oligosaccharide to an oligosaccharide acceptor containing an N-acetylglucosamine moiety, i.e. an intermediate oligosaccharide. This intermediate oligosaccharide is added to the culture medium wherein a microbial cell comprising an α-1,3/4-fucosidase activity is grown. The donor oligosaccharide is either synthesized by the cell or also added to the culture medium and internalized into the cell.

In a preferred embodiment, the α-1,3/4-fucosidase is AfcB from *Bifidobacterium bifidum* and the donor substrate is 3-fucosyllactose. The hydrolase activity of the enzyme AfcB is abolished by protein engineering (Zeuner, B. et al. (2018). Loop engineering of an α-1,3/4-I-fucosidase for improved synthesis of human milk oligosaccharides. Enzyme and microbial technology, 115, 37-44.). The product to be obtained from the intracellular transglycosilation reaction on the internalized intermediate oligosaccharide is an α-1,3-fucosylated or α-1,4-fucosylated oligosaccharide. Fucosylated oligosaccharide products can be obtained from the internalized acceptor substrate when using engineered AfcB and 3-FL as donor substrate: 1) LNFP-II, when the intermediate oligosaccharide is LNT, 2) LNFP-III, when the intermediate oligosaccharide is LNnT, 3) LNDFH-1, when the intermediate oligosaccharide is LNFP-I.

When using transglycosidases for the elongation of an oligosaccharide the donor substrate is a di- or oligosaccharide. The donor substrate can either be synthesized by the genetically modified cell or internalized from an extracellular source. Uptake of the donor substrate can be passive by diffusion or intake of the donor substrate can be achieved either by a transport system that is endogenous to the host cell or by a transport system that is recombinant to the host cell. Such that sugar transport system can be found in but not limited to the major facilitator superfamily, comprising secondary transport mechanisms, or ATP depended transport systems (ABC transporters).

The genetically engineered microbial cell may either be a prokaryotic cell or a eukaryotic cell. Non-limiting examples of genetically engineered eukaryotic cells for the production of a desired oligosacchairde by the porcess disclosed herein are yeast cells. The yeast cell may be selected from the group of genera consisting of Saccharomyces such as *Saccharomyces cerevisiae,* Schizosaccharomyces, Pichia, Hanensula and Yarrowia. The genetically engineered prokaryotic cell may be a cell selected from group of genera consisting of Escherichia, Corynebacterium, Bacillus, Lactobacillus, Lactococcus and Pseudomonas. Particularly suitable prokaryotic cells for the production of the desired oligosaccharide are *Escherichia coli, Corynebacterium glutamicum* and *Bacillus subtilis.*

The genetically engineered microbial cell comprises one or more exogenous functional genes. Exogenous functional genes are transcribed either from extrachromosomal vectors or they are integrated into the genome of the genetically engineered microbial cell. Transcription of these functional genes is either constitutive or inducible, e.g. by addition of an external inducer or by induction of a metabolite synthesized by the cell. A person skilled of the art knows how to configure nucleotide sequences which constitute a functional gene comprising a promoter, optionally an operator, a ribosomal binding site, the protein coding sequence of interest and a transcriptional terminator in order to obtain constitutive or inducible recombinant expression.

Biosynthesis of enzymes encoded by endogenous genes can be modified by genetically modifying the microbial cell, e.g. leading to an enhanced or reduced expression. Genetic modifications comprise but are not limited to modifying the transcriptional promotor and operator sequences, modifying the ribosome binding site, modifying the codon usage, or modifying the copy number of the gene or genes of interest in the cell. The modification of functional nucleotide sequences can either result in an overexpression of the gene or the genes of interest or in a more balanced expression of different genes to obtain an optimal amount of the nucleotide activated sugar donor substrate for the synthesis of the desired oligosaccharide.

Genetic modification to obtain an overexpression of a gene of interest means that the expression of the gene of interest is higher than in the wild-type cell. The genetic modification increases the expression of the gene of interest by 10%, 20%, 30%, 40% or 50%. Preferred the expression is increased 2-fold or up to 10-fold compared to the expression in the wild-type. The overexpression can be constitutive or inducible by addition of an external inducer or by induction of a metabolite synthesized by the cell.

The genetically engineered microbial cell does not contain any enzymatic activity lyable to degrade the acceptor substrate internalized into the cell or in case of using the transglucosylation reaction the donor substrate taken up by the cell or synthesized by the cell.

In the process of producing a human milk oligosaccharide according to the process disclosed herein, a genetically engineered microbial cell is cultivated, said genetically engineered microbial cell may possess:
- at least one functional gene encoding a glycosyltransferase, and
- the genes enabling the cell to synthesize one or more nucleotide activated sugar(s) as donor substrate(s) or the genes encoding enzymes capable to synthesize a nucleotide activated sugar from a monosaccharide precursor; and/or
- at least one functional gene encoding an enzyme with transglycosidase activity, and functional genes to allow the microbial cell to take up oligosaccharides (di- or trisaccharides) to be used as donor substrates in the transglycosidase reaction; and
- at least one functional gene encoding an oligosaccharide transport system to facilitate the up-take of oligosaccharides with three or more monosaccharide units which shall serve as intermediate oligosaccharide in the glycosyltransferase or transglycosidase reaction to obtain a desired oligosaccharide with four, five or more monosaccharide units.

Said genetically engineered microbial cell does not contain any enzyme activity liable to degrade the acceptor substrate being internalized into the cell or in case of using the transglycosylation reaction the donor substrate taken up by the cell or synthesized by the cell.

The process comprises cultivating the genetically engineered microbial cell for the production of a desired oligosaccharide in a culture medium which contains the intermediate oligosaccharide for the the genetically engineered microbial cell to take up the intermediate oligosacchride and to transfer at least one monosacchride moiety from a donor substrate to the intermediate oligosaccharide, thereby synthesizing the desired oligosaccharide.

Cultivating said genetically engineered microbial cell means growing the microbial cells in a culture medium which contains a carbon source and the intermediate oligosaccharide. The culture medium may contain additional compounds. If a monosaccharide moiety is transfereed to the intermediate oligosaccharide by means of a a a transglycosidase, the donor substrate for the monosaccharide moiety can be added to - and thus also be contained in - the culture medium. The carbon source may be selected from the group consisting of glycerol, glucose, fructose, sucrose, cellulose, glycogen, lactose, or more complex substrates such as molasse and/or corn-syrup. Preferably the carbon source is selected from at least one of the group consisting of glycerol, glucose, fructose, sucrose.

Cultivating the microbial cells can either be performed as a batch fermentation, as a fed-batch fermentation or in a continues fermentation manner. Preferably, the cultivating is performed as a fed-batch fermentation comprising a phase of exponentially growth of the microbial cells with the carbon source in excess and a subsequent phase of cell growth that is limited by the availability of the carbon source. More preferably is a continuous sequential fermentation process involving defined compartments for production of the intermediate oligosaccharide, production of the desired olgiosaccharide, and/or degradation of undesired oligosaccharide by-products.

The desired oliogsaccharide is retrieved from the culture medium and/or the microbial cell for porducing the desired oligosaccharide at the end of the last fermentation step, i.e. the fermentation step in which the desired oligosaccharide is synthesized by the microbial cell.

Retreiving the desired oligosaccharide may include various retrieval steps including, but not limited to, centrifugation, microfiltration, ultrafiltration, nanofiltration, ion exchange treatment (cation exchange treatment and/or anion exchange treatment), electrodialysis, reverse osmosis, solvent evaporation, crystallization, spray drying and/or active carbon treatment. It is understood that - in a first retrieval step - the microbial cells are separated from the culture medium. This separation may be obtained by cetrifugation and/or microfiltration and ultrafiltration steps.

The desired oligosaccharide can be obtained from the microbial cells by disrupting the microbial cells producing the desired oligosaccharide and retrieving the desired oligosaccharide from the cytoplasmic fraction of the microbial cells.

In a preferred embodiment, the microbial cells producing the desired oligosaccharide secrete said oligosaccharide into the culture medium. The microbial cells are then separated from the culture medium at the end of the fermentation step by centrifugation and/or filtration, and the desired oligosaccharide is retrieved from the cell-free culture medium.

Secretion of the desired oligosaccharide may occur either by passive diffusion across the cell membrane or by active transport processes. The functional genes encoding proteins facilitating active export processes of the desired oligosaccharide or nucleotide sequences equivalent to the functional genes may be either endogenous to the genetically engineered microbial cell or recombinant. In case the functional genes or nucleotide sequences equivalent thereto are recombinant, they may be expressed in the microbial cell either from the genomically integrated copies (by integration of an expression fragment (operon) into the genome) or from an extrachromosomal vector.

For or during retrieval of the desired oligosaccharide, the culture medium or intracellular fraction can be subjected to a sequential series of filtration steps to i) separate the biomass from the culture medium, e.g. by microfiltration and ultrafiltration; to ii) remove small molecules such as water, monosaccharides or salts, e.g. by nanofiltration; to iii) concentrate the oligosaccharide-containing solution, e.g. by reverse osmosis or evaporation. The concentrate can be subjected to further purification steps, e.g. by subjecting the concentrate to treatment on activated carbon. If necessary, the desired oligosaccharide can be deionized by electrodialysis, and/or filtered for removal of endotoxins and microorganisms accomplished during processing of the desired oligosaccharide in the retrieval process to obtain a product with very low microbial load.

The retrieved/purified desired oligosaccharide can be stored and used in liquid form or as dried substance. To obtain the desired oligosaccharide in solid form it can be freeze-dried, spray-dried, granulated, crystallized, dried on a roller-dryer or a belt-dryer.

If the retrieved/purified desired oligosaccharide is a human milk oligosaccharide, it can be used as supplement in infant formula, toddler formula, infant cereals, functional food being served as a drink, a bar, a yoghurt or the like, medical nutrition, or general food.

The desired oligosaccharide can either be used separately or in combination with other oligosaccharides. These other oligosaccharides can also belong to the group of human milk oligosaccharides or to other oligosaccharides such as galactooligosaccharides, maltodextrin, or fructooligosaccharides.

The desired oligosaccharide or the combination of the desired oligosaccharide with other oligosaccharides can be used in any one of above referenced foods in combination with probiotic bacteria. Preferably, said probiotic bacteria are bacterial strains of one or more of the genera of Bifidobacteria and Lactobacilli.

Human milk oligosaccharides are known to elaborate a wide range of positive effects on the development of neonates but also on the health of adults. Human milk oligosaccharides are prebiotics, thus favoring the development of a healthy microbiome in the neonate gut (Chu, D. M., & Aagaard, K. M. (2016). Microbiome: Eating for trillions. Nature, 532(7599), 316.). In addition, these oligosaccharides were shown to reduce the risk of infectious diseases caused by bacterial or viral pathogens (Craft, K. M., & Townsend, S. D. (2017). The human milk glycome as a defense against infectious diseases: rationale, challenges, and opportunities. ACS infectious diseases, 4(2), 77-83.) and act directly antimicrobial (Craft, K. M., & Townsend, S. D. (2019). Mother Knows Best: Deciphering the Antibacterial Properties of Human Milk Oligosaccharides. Accounts of chemical research, 52(3), 760-768.). Sialylated oligosaccharides were found to supply the developing brain with sialic acid (Wang, B. (2009). Sialic acid is an essential nutrient for brain development and cognition. Annual review of nutrition, 29, 177-222., Mudd, A. et al. (2017). Dietary sialyllactose influences sialic acid concentrations in the prefrontal cortex and magnetic resonance imaging measures in corpus callosum of young pigs. Nutrients, 9(12), 1297.). In adults human milk oligosaccharides were shown to improve the gut microbiota and metabolite composition in IBS patients (Jackson, F. et al. (2018). Human Milk Oligosaccharides Are Able to Impact the Gut Microbiota and Metabolites in Irritable Bowel Syndrome Patients: 2788. American Journal of Gastroenterology, 113, S1548.).

The desired oligosaccharide or combinations of the desired oligosaccharide with other oligosaccharides and/or with probiotics can be applied as food supplement for the prevention of dysbiosis, for the prevention of specific infectious diseases or to strengthen the immune system in general.

The intermediate oligosaccharide may be obtained by chemical syntheiss or biocatalysis using purified or enriched enzyme preparations or crude cell extracts of cells synthesizing appropriate enzymes. In a preferred embodiment, the intermediate oligosaccharide is obtained by microbial fermentation using a genetically engineered microbial cell for the production of the intermediate oligosaccharide from a simple carbon source, a disaccharide or another oligosaccharide.

In such embodiments where sequential fermentation steps are used, different genetically engineered microbial cells are used for producing the intermediate oligosaccharide(s) and the desired oligosaccharide. For example an intermediate oligosaccharide may be produced by a genetically engineered yeast cell while the desired oligosaccharide is produced from the intermediate oligosaccharide by a genetically engineered bacterial cell, or vice versa.

In some embodiments, the intermediate oligosaccharide is at least partially purified from the culture medium in that the culture medium at the end of the fermentation step to provide the intermediate oligosaccharide is subjected to one or more purification steps. Said at least one purification step for purifying the intermediate oligosaccharide may be selected from the group consisting of centrifugation, microfiltration, ultrafiltration, nanofiltration, ion exchange treatment (cation exchange treatment and/or anion exchange treatment), electrodialysis, reverse osmosis, solvent evaporation, crystallization, spray drying and/or active carbon treatment.

It is understood that at least the microbial cells for producing the intermediate oligosaccharide are removed from the culture medium containing the intermediate oligosaccharide prior to using the thus obtained cell free culture medium containing the intermediate oligosaccharide as culture medium for the cultivation of the genetically engineered microbial cell for the production of the desired oligosaccharide.

The intermediate oligosaccharide obtained from one fermentation step can be transferred to the following fermentation step by using i) the culture medium at the end of the fermentation step and after removal of the microbial cells; ii) as a process stream wherein the intermediate oligosaccharide is enriched as compared to the culture medium, for example in that the cell-free clture medium containing the intermediate oligosaccharide is subjected to at least one desalting step and/or at least one decolorating step; and/or iii) by adding a purified intermediate oligosaccharide either as concentrate or as solid material.

Intermediate oligosaccharide that is not converted to the desired oligosaccharide during the last fermentation step of the process can be degraded by the addition of enzymes exhibiting specific hydrolytic activity on the remaining intermediate oligosaccharide and/or on degradation products of the intermediate oligosaccharide, but not on the desired oligosaccharide. These enzymes exhibiting said specific hydrolytic activity can be added as pure enzymes, crude extracts of cells expressing said enzymes or by addition of a second set of microbial cells which synthesize such enzymes and preferably secrete these enzymes exhibiting said specific hydrolytic activity into the culture medium. Retrieval and purification of the desired oligosaccharide from the culture medium is facilitated by the hydrolytic degradation of remaining intermediate oligosaccharides since the desired oligosaccharide and the intermediate oligosaccharide differ in only one or two monosaccharide moietise from each other, and are therefore difficult to separate from eac other by technical means in industrial scale at reasonable costs.

In preferred embodiment, the process according to the invention includes a first fermentation step for the production of an intermediate oligosaccharide. The genetically engineered microbial cell used to produce this intermediate oligosaccharide comprises a glycosyltransferase to transfer a monosaccharide moiety from a donor substrate to an acceptor substrate. The donor substrate may be a nucleotide-activated sugar as described herein before. The acceptor substrate is lactose. The lactose is added to the culture medium and is taken up by the respective microbial cell to convert the lactose into an intermediate oligosaccharide consisting of at least three monosaccharide units.

In another embodiment, the acceptor substate lactose is synthesized by the genetically engineered microbial cell being used in the first fermentation step. Said microbial cell comprises a β-1,4-galactosyltransferase specifically transferring a galactose molecule onto a glucose molecule. The microbial cell further contains a second glycosyltransferase using the intracellularly generated lactose as acceptor substrate to synthesize an intermediate oligosaccharide of three monosaccharide units. The culture medium used for cultivating this microbial cell may contain sucrose, glucose or a combination of glycerol and glucose as carbon source. Said microbial cell may have been engineered such that enzymes converting the glucose into glucose-6-phosphate have been inactivated or deleted from the genome of the microbial cell.

The intermediate oligosaccharide can be added to the culture medium for cultivating the microbial cell for the production of the desired oligosaccharide i) once during the fermentation step, ii) as bolus' several times during the fermentation step or iii) in a continuous manner throughout the fermentation step.

The process may comprise splitting the synthesis of a desired oligosaccharide into at least two separate fermentation steps, each utilizing a different genetically engineered microbial cell which possesses the enzymatic activities that are required for the synthesis of the oligosaccharide to be obtained in that specific fermentation step.

In contrast to direct production of the desired oligosaccharide using a single genetically modified cell, intermediate oligosaccharides containing at least one monosaccharide moiety less than the desired oligosaccharide are added to the culture medium as precursors, taken up by the genetically engineered microbial cell and are converted to the desired oligosaccharide by being extended by one or more monosaccharide units.

The process of sequential fermentation of a desired oligosaccharide with four, five or more monosaccharide units comprises two or more fermentation steps. In a first fermentation step, a genetically engineered microbial cell is cultivated to produce an intermediate oligosaccharide comprising three or more monosaccharide units using an exogenous acceptor substrate such as lactose. This microbial cell preferably secrets the intermediate oligosaccharide into the culture medium. The intermediate oligosaccharide can be - at least partially - purified from the culture medium, it can be enriched by removal of contaminating substances, particularly other carbohydrates, or the culture medium containing the intermediate oligosaccharide can directly be used for the second cultivation step. In the second fermentation step, a second genetically engineered microbial cell is cultivated in a culture medium containing the intermediate oligosaccharide to be extended by one or more monosaccharide units by the microbial cell. In an optional third fermentation step the oligosaccharide obtained from the second fermentation can be applied as precursor to yet another genetically engineered microbial cell that is able to take up that oligosaccharide and modify that oligosaccharide by adding one more monosaccharide unit.

In a specific embodiment of the invention the desired oligosaccharide is a triose and the intermediate oligosaccharide is lactose that is produced by an organism able to synthesize lactose using a specific galactosyltransferase linking a galactose unit to glucose that is added to the fermentation process. The glucose added can either serve as well as c-source and substrate for the galactosyltransferase reaction or the glucose can be added to the fermentation medium in addition to another c-source or the glucose used as substrate for the enzymatic galactosyltransferase reaction can be achieved by conversion of another enzymatic reaction such as a sucrose hydrolase when feeding sucrose as carbon source.

In other embodiments, two different intermediate oligosaccharides are produced by using different genetically engineered microbial cells in separate fermentation steps. Any one of both of these intermediate oligosaccharides is/are either purified from its culture medium or enriched from its culture medium, or the culture media are directly used for a third fermentation step, wherein a third genetically engineered microbial cell produces the desired oligosaccharide using the two intermediate oligosaccharides.

In another embodiment of the invention the separate steps of oligosaccharide production are not conducted in spatially separated fermentation vessels but in a single vessel that is separated into two compartments by a semipermeable membrane. The characteristics of said semipermeable membrane allows the flux of the intermediate oligosaccharide produced by a first genetically engineered microbial cell in one of the two compartments into the other compartment, but does not allow flux of the more complex oligosaccharide produced by the second genetically engineered microbial cell. Thus e.g. the microbial cell grown in one compartment is able to convert the exogenous acceptor lactose that is added to the fermentation vessel to the intermediate oligosaccharide comprising three monosaccharide units. This trisaccharide passes the semipermeable membrane, is taken up by the second microbial cell growing in the second compartment, and is converted to a more complex oligosaccharide comprising at least four monosaccharides.

In an embodiment, the desired oligosaccharide comprising four or more monosaccharide units is a tetraose. The genetically engineered microbial cell comprises a β-1,3-galactosyltransferase or a β-1,4-galactosyltransferase. In addition, the genetically engineered microbial cell is to produce UDP-galactose. The intermediate oligosaccharide is a triose that is internalized by the genetically engineered microbial cell and galactosylated to obtain the tetraose. In a preferred embodiment the intermediate oligosaccharide is LNT II and the desired oligosaccharide is lacto-N-tetraose (LNT) or lacto-N-neotetraose (LNnT), when the microbial cell possesses the β-1,3-galactosyltransferase or the β-1,4-galactosyltransferase, respectively.

In another embodiment, the desired oligosaccharide is a fucosylated oligosaccharide consisting of at least five monosaccharide moieties. The genetically engineered microbial cell possesses an α-1,2-fucosyltransferase, an α-1,3-fucosyltransferase or an α-1,3/4-fucosyltransferase. In addition, the genetically engineered microbial cell is able to synthesize GDP-fucose. The intermediate oligosaccharide that is taken up by the genetically engineered microbial cell is a tetraose. When providing LNT as intermediate oligosaccharide and the genetically engineered microbial cell possesses an α-1,2-fucosyltransferase, the desired oligosaccharide produced is LNFP-I. If the genetically engineered microbial cell possesses an α-1,3/4-fucosyltransferase, the oligosaccharides being produced are LNFP-II or LNFP-V. If the genetically engineered microbial cell possesses an α-1,3-fucosyltransferase, the desired oligosaccharide is LNFP-V.

When providing LNnT as intermediate oligosaccharide the desired oligosaccharides being obtained are LNnFP-I or LNFP-III provided that the genetically engineered microbial cell possesses an α-1,2-fucosyltransferase or an α-1,3/4-fucosyltransferase, respectively.

In another embodiment, the desired oligosaccharide is a hexasaccharide. The genetically engineered microbial cell producing the hexasaccharide possesses two glycosyltransferase genes, an α-1,2 fructosyltransferase and an α-1,3-N-acetylgalactosaminyl transferase. The genetically engineered microbial cell is able to synthesize GDP-fucose and UDP-N-acetylgalactosamine. The desired oligosaccharide is the human blood group antigen (HBGA) A type 1 when LNT is provided as intermediate oligosaccharide or HBGA A type 2 when LNnT is provided as intermediate oligosaccharide.

In an alternative embodiment to produce the HBGA A type 1 and A type 2 glycans, the genetically engineered microbial cell possesses an α-1,3 N-acetylgalactosaminyl transferase, is able to synthesize UDP-N-acetylgalactosamine and to internalize LNFP I or LNnFP I as intermediate oligosaccharide. Accordingly, when the desired oligosaccharide is HBGA B type 1 or HBGA B type 2 the intermediate oligosaccharide provided is LNFP I or LNnFP I. The genetically engineered microbial cell used to modify LNFP I or LNnFP I as the intermediate oligosaccharide possesses an α-1,3 galactosyltransferase and is able to synthesize UDP-galactose as donor substrate.

In yet another embodiment, the desired oligosaccharide is lacto-N-hexaose (LNH). A first intermediate oligosaccharide obtained by a first fermentation step is LNT II. Said LNT II is then provided to the second fermentation step. The genetically engineered microbial cell used as processing aid in the second fermentation step comprises a β-1,3-galactosyltransferase and is able to synthesize UDP-galactose. The second fermentation step leads to LNT that is/can be provided in a third fermentation step to be enlarged by one N-acetylglucosamine moiety using a genetically engineered microbial cell comprising a α-1,6 N-acetylglucosaminyl transferase and being able to synthesize UDP-N-acetylglucosamine. In a fourth fermentation step, another genetically engineered microbial cell comprising a β-1,4-galactosyltransferase and being able to synthesize UDP-galactose is/can be used to produce the desired oligosaccharide LNH. Producing LNH in a single fermentation step from lactose by using a genetically engineered microbial cell that comprises all enzymes and metabolic pathways mentioned in this paragraph herein before is not possible due to unspecific side reactions of the galactosyltransferases resulting in LNT and LNnT as intermediates that are than subjected to various elongations by the α-1,3, and α-1,6 N-acetylglucosaminyltransferases. Thus, such a production of LNH from lactose by a single genetically engineered microbial cell would lead to substantial amounts of undesired oligosaccharides which in turn would constitute impurities in the desired LNH product affecting yield and purity of said LNH.

In additional and/or alternative embodiments, the desired oligosaccharide comprises at least one sialic acid moiety. The intermediate oligosaccharide that is added to the genetically engineered microbial cell to be internalized and sialylated comprises at least four monosaccharide moieties. Preferably, the intermediate oligosaccharide is LNT to obtain lactosialyltetraose a or b (LSTa or LST b), or LNnT to obtain LST c. The genetically engineered microbial cell to produce one of these desired oligosaccharides possesses an α-2,3 sialyltransferase or an α-2,6 sialyltransferase and is able to synthesizes CMP-N-acetylneuraminic acid.

In additional and/or alternative embodiments, the desired oligosaccharide comprises a fucose moiety and a sialic acid moiety. When the intermediate oligosaccharide being provided to the genetically engineered microbial cell to be internalized is 3-fucosyllactose, then fucosyl-sialyllactose is produced as the desired oligosaccharide as the genetically engineered microbial cell possesses an α-2,3 sialyltransferase and is able to synthesize CMP-N-acetylneuraminic acid.

Alternatively, fucosyl-sialyllactose is produced as desired oligosaccharide by providing 3-sialyllactose as intermediate oligosaccharide to a genetically engineered microbial cell that possesses an α-1,3-fucosyltransferase and is able to synthesize GDP-fucose.

Additional embodiments for the production of different desired oligosaccharides comprising four, five or more monosaccharide moieties from an internalized intermediate oligosaccharide are shown in Table 1. Suitable donor substrates and enzymes for the transfer of a monosaccharide moiety from the donor substrate to the intermediate oligosaccharide can be inferred from the present disclosure even if not explicitly identified in Table 1 for each of the embodiments.

The present invention will be described with respect to particular embodiments and with reference to drawings, but the invention is not limited thereto but only by the claims. Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description and drawings provided herein, numerous specific details are set forth. However, it is understood that embodiments of the invention may be practiced without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

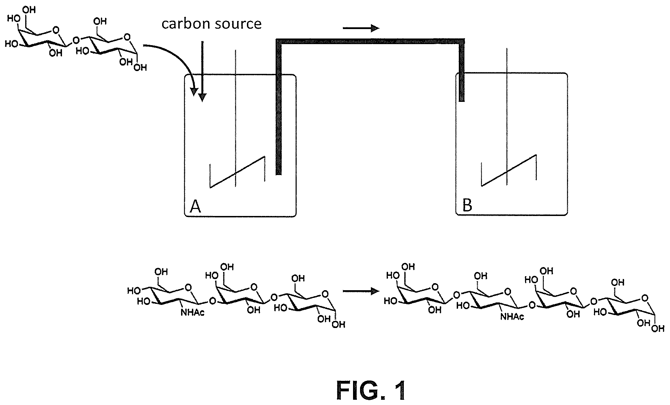

Referring now to FIG. 1, the principle of sequential fermentation is schematically illustrated with respect to a process for the production of LNnT, the process comprising two fermentation steps. A first fermentation step is performed in a first fermentation vessel (A). In the first fermentation step, a genetically engineered microbial cell (not shown) is provided which possess a β-1,3-N-acetylglucosaminyltransferase and is able to synthesize UDP-N-acetylglucosamine in a culture medium. The genetically engineered microbial cell is grown on a carbon source such as e.g. glucose, fructose, glycerol or sucrose. The genetically engineered microbial cell is cultivated in the presence of lactose as an acceptor substrate for an N-acetylglucosamine moiety. The microbial cells growing in the first fermentation vessel take up the acceptor substrate and convert it to an intermediate oligosaccharide such as lacto-N-triose II. The intermediate oligosaccharide is transferred from the first fermentation vessel into a second fermentation vessel (B). Another genetically engineered microbial cell is cultivated in the second fermentation vessel. It is understood that said another genetically engineered microbial cell is also grown on a carbon source such as e.g. glucose, fructose, glycerol or sucrose. Said another genetically engineered microbial cell internalizes the intermediate oligosaccharide LNT II, possesses a β-1,4-galactosyltransferase, is able to synthesize UDP-galactose, and thereby converts LNT II to the desired oligosaccharide lacto-N-neotetraose. The desired LNnT may be retrieved from the culture medium of the second fermentation vessel and/or the latter genetically engineered microbial cell.

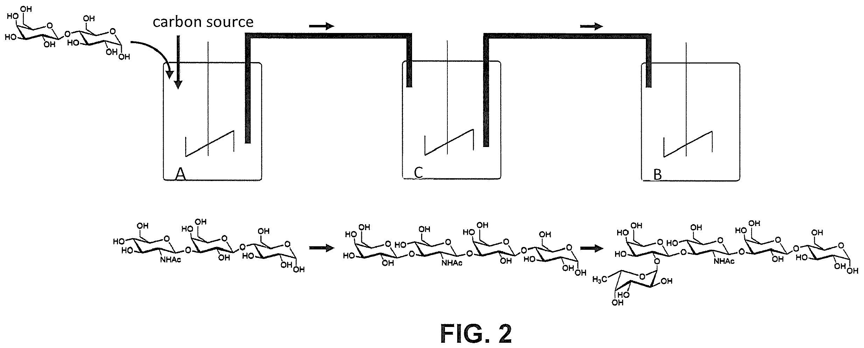

FIG. 2 illustrates another embodiment of the sequential fermentation. The process according to this embodiment comprises three fermentation steps for the production of a pentasaccharide, e.g. LNFP I. In this embodiment, the sequential fermentation process illustrated in FIG. 1 is expanded by an additional fermentation step, wherein the desired oligosaccharide pursuant to the embodiment according to FIG. 1 is used as a second intermediate oligosaccharide that is provided to a third fermentation step in another fermentation vessel (C). A genetically engineered microbial cell is cultivated in the third fermentation step. Said genetically engineered microbial cell internalizes LNnT that is provided to the microbial cell during the third fermentation step. The genetically engineered microbial cell possesses an α-1,2-fucosyltransferase and is able to synthesize GDP-fucose, thereby converting LNnT to LNFP I as the desired oligosaccharide in this embodiment The desired LNFP I may be retrieved from the culture medium of the second fermentation vessel and/or the latter genetically engineered microbial cell.

The process of sequential fermentation for the production of a desired oligosaccharide comprises at least two fermentation steps. An intermediate oligosaccharide is produced in an initial fermentation step, and the desried oligosacchride is produced in a second or the last fermentation step of the sequential fermentation process.

The at least two fermentation steps can be performed spatially and/or temporally separated from each other. That said, it is understood that the first and at least one of the one or more subsequent fermentation steps can be performed in separate, i.e. individual, fermentation vessel in that the intermediate oligosaccharide(s) produced in the first and optionally additional fermentation steps is transferred to another fermentation vessel for the subseequent production of another intermediated oligosaccharide or the desired oligosaccharide. In is also understood that it is preferred that none of the genetically engineered microbial cells being employed in one fermentation step are transferred to the subsequent fermentation step.

Alternatively, it is also possible to use the same fermentation vessel for all fermentation steps of the sequential fermentation process. Using a single fermentation vessel for the fermentation steps of the sequential fermentation process requires removal of the entire content of the fermentation vessel after a fermentation step is completed, sanitizing the fermentation vessel and providing all elements for the subsequent fermentation step to the sanitized fermentation vessel.

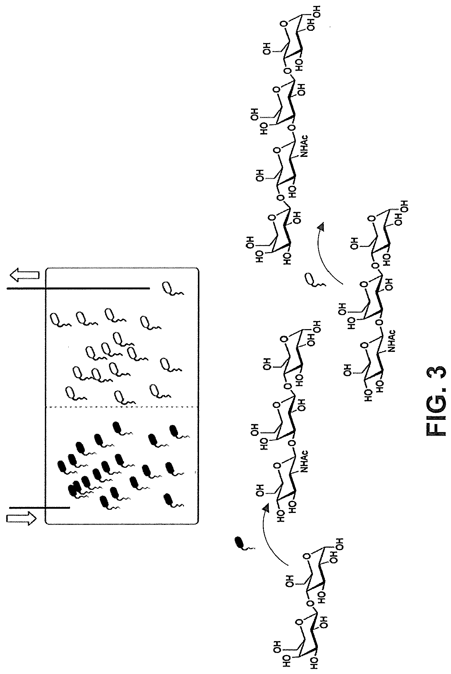

In another embodiment, the at least two fermentation steps can be performed spatially separated from each other but at the same time in a single fermentation vessel. FIG. 3 illustrates the principle of sequential fermentation being conducted in one fermentation vessel comprising two spatially separated compartment for cultivating two different genetically engineered microbila cells. The two compartments within a single fermentation vessel are sepqrated from each other by The semipermeable membrane prevents a transfer of the different genetically engineered microbial cells from their compartment to the other compartment, but permits streaming of the intermediate oligosaccharide from the compartment in which it was produced to the other compartment where it is used. Preferably, the semipermeable membrane also prevents streaming of the desired oligosaccharide from the compartment in which it was produced to the compartment in which the intermediate oligosaccharide was generated. The differnet genetically engineered microbial cells within the different compartments posess different enzymes which enable the microbial cells to convert different oligosaccharides. For example, the process of sequential fermentation using simultaneously performing two fermentation steps in a single fermentation vessel can be used for the production of LNT or LNnT in that lactose is converted lacto-N-triose II by a first population of genetically engineered microbial cells (shown as black flagellated cells) and streaming of said lacto-N-triose II as intermediate oligosaccharide across the semipermeable membrane (shown as dotted line in the fermentation vessel) into the other compartment where LNT II is converted to lacto-N-tetraose by a different population of another genetically engineered microbial cell (shown as white flagellated cells).

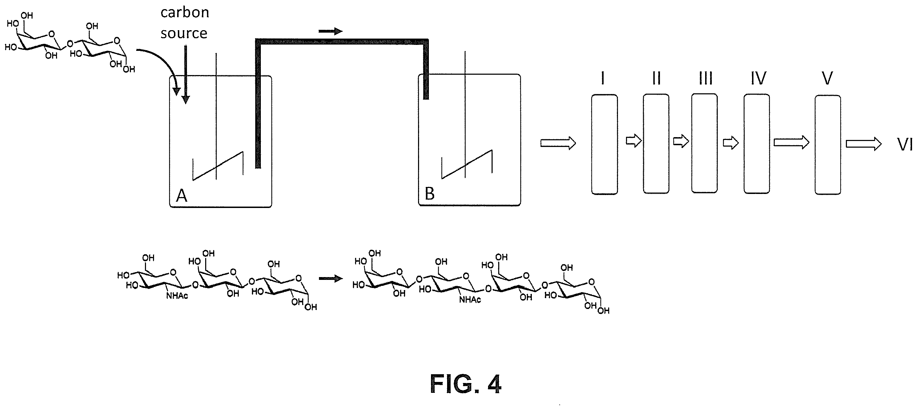

In additional embodiments, e.g. illustrated in FIG. 4, is the desired oligosaccharide being produced in the sequential fermentation process subjected directly to retrieval proceedings which involve multiple retrieval steps including centrifugation, microfiltration, ultrafiltration, nanofiltration, ion exchange treatments, electrodialysis , reverse osmosis, solvent evaporation, drying and the like. FIG. 4 illustrates the embodiment with respect to the production of LNnT as desired oligosaccharide. At the end of fermentation step (B) to produce the desired oligosaccharide LNnT from LNT II (see FIG. 1), the culture medium containing the desired oligosaccharide is subjected to microfiltration (I) and ultrafiltration (II) for the removal of cells from the culture medium. Subsequently, small molecules such as water, monosaccharides and salts are removed from the cell-free culture medium by nanofitration (III) to obtain a oligosaccharide containing process stream. In a furhter retrival step the oligosaccharide containing process stream is concentrated by using reverse osmosis or evaporation (IV). A further purification step is performed by contacting the concentrated process stream to treatment with activated carbon (V). The thus obtained process stream containing the desired oligosaccharide may optionally be deionized by using electrodialysis and again concentrated using reverse osmosis or evaporation, filtered for removal of endotoxins. At the end of the retrival process, the desired oligosaccharide product may be dried to obtain the desired oligosaccharide in form of a powder product (VI).

In summary, the process for the production of a desired oligosaccharide as described herein is advantageous in that it allows to minimize the presence of undesired by-products in the preparation of the desired oligosaccharide which occur due to side reactions and competing reactions (e.g. galactosylation of lactose by a β1-4-galactosyltransferase used in the conversion of Lacto-N-triose II to Lacto-N-neotetraose, or the transfer of an additional glucosamine residue to Lacto-N-neotetraose by a promiscuous glucosamyltransferase) as the resulting oligosaccharide by-products are highly undesired, because they are chemically very closely related to the desired oligosaccharide and are therefore problematic and thus costly to separate away from the desired oligosaccharide.

### Examples

### Example 1: LC/MS analytics used for identification of oligosaccharides produced by fermentation of bacterial strains:

Molecular identity of LNFP-II and LNFP-III produced by bacterial strain fermentation was confirmed by MRM (multiple reaction monitoring) using a LCMS-8050 system. The LCMS system incorporated a Nexera X2 SIL-30ACMP autosampler run at 8°C, a LC-20AD HPLC pump, a CTO-20AC column oven run at 35°C and a Triple-Quadrupole (QQQ) mass analyzer (all parts of the LCMS system were purchased by Shimadzu Corporation, Kyoto, Japan). Prior to LCMS analysis culture supernatants and intracellular extracts were purified by filtration (0.22 µm pore size) following solid phase extraction (SPE) on an ion exchange matrix (Strata ABW; Phenomenex, Aschaffenburg, Germany). For each analytical run a sample volume of 1 µl was injected into the instrument. Chromatographic separation was performed using a XBridge Amide HPLC column (3.5 µm, 2.1 Å 50 mm; Waters, Eschborn, Germany) considering 5 min isocratic elution (60% acetonitrile with 0.1% (v/v) NH₄OH) with a flow rate of 300 µl/min. Eluting compounds were directly introduced to the Triple-Quadrupole mass analyzer considering Electro spray ionization (ESI). Thereby, oligosaccharide precursor ions were selected in the instruments quadrupole 1 (Q1) and fragmented in the collision cell (Q2) using argon as collision gas following selection of fragment ions in quadrupole 3 (Q3). MRM analysis was conducted in ESI negative ionization mode, whereby the instrument was operated at unit resolution. Collision energy, Q1 and Q3 pre-bias were optimized for LNT II, LNnT, LNFP-II and III individually. The details on selected MRM transitions and collision energies (CE) are provided in Table 2.

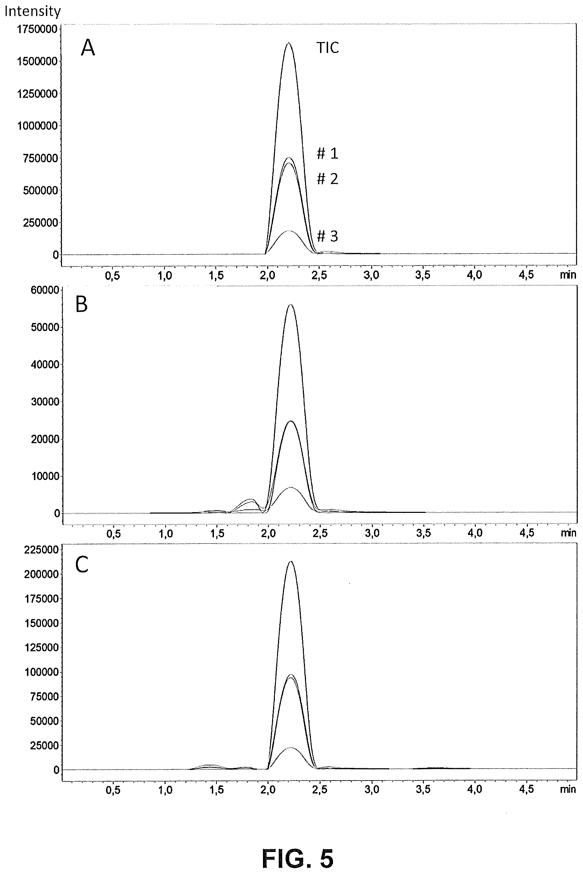

To support identity of LNT II, LNnT, LNFP-II and LNFP-III originating from sequential fermentation processes, the corresponding MRM profiles and retention times of these desired oligosaccharides were compared with the MRM profiles of commercially available substance standards (Dextra Laboratories Ltd., Reading, PA, USA) (FIGs 5 to 8).

**Table 2: List of MRM transitions for identification of LNT II, LNnT, LNFP-II and LNFP-III in negative ion mode (CE = Collision energy) of the Shimadzu LCMS-8050 Triple Quadrupole (QQQ) Mass Analyzer.**

| Metabolite | Transition 1 [m/z Q1>Q3] | CE | Transition 2 [m/z Q1>Q3] | CE | Transition 3 [m/z Q1>Q3] | CE |
|---|---|---|---|---|---|---|
| LNT-II [M-H]⁻ | 544,20>161,00 | 16 | 544,20>382,10 | 11 | 544,20>112,90 | 28 |
| LNnT [M-H]⁻ | 706,10>179,20 | 29 | 706,20>263.25 | 21 | 706,20>382,30 | 17 |
| LNFP-II [M-H]⁻ | 852,30>348,20 | 23 | 852,30>163,05 | 40 | 852,30>288,20 | 29 |
| LNFP-III [M-H]⁻ | 852,30>364,10 | 21 | 852,30>179,10 | 35 | 852,30>161,15 | 30 |

FIG. 5 indicates the identity of LNFP-III produced in a sequential fermentation process described herein by LCMS based MRM analysis. Chart A shows the MRM profile of commercially available LNFP-III standard (Dextra Laboratories Ltd., Reading, PA, USA) displaying diagnostic transitions: TIC (Total ion current); #1 = [M-H]⁻ 852,30>364,10 m/z, CE=21; #2 = [M-H]⁻ 852,30>179,10 m/z, CE=35; #3 = [M-H]⁻ 706,20>382,30 m/z, CE=30. Chart B shows the corresponding MRM profile of LNFP-III produced by sequential fermentation and retrieved from the culture medium of the last fermentation step. Chart C shows the corresponding MRM profile of LNFP-III produced by the sequential fermentation and retrieved from intracellular fraction of the genetically engineered microbial cells producing the LNFP-III as desired oligosaccharide.

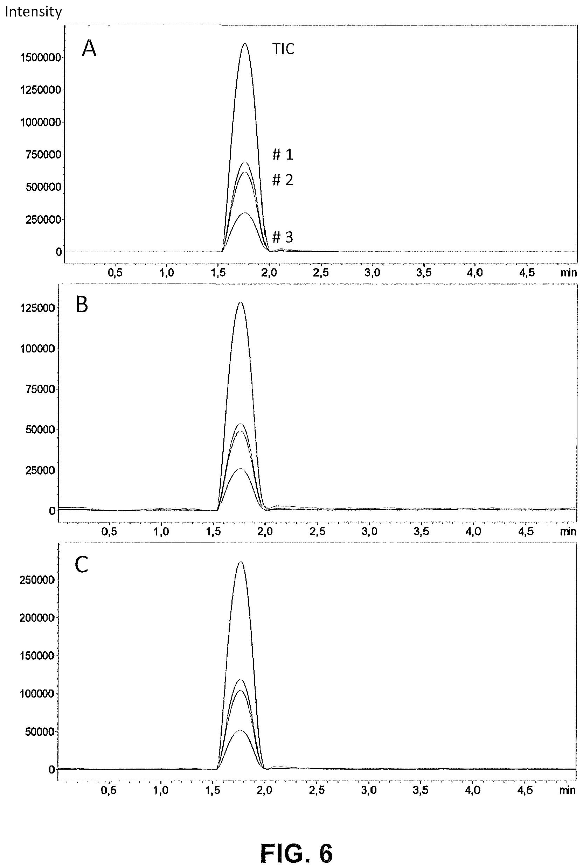

FIG. 6 indicates the identity of LNnT produced in a sequential fermentation process described herein by LCMS based MRM analysis. Chart A shows the MRM profile of commercially available LNnT standard (Carbosynth, Berkshire, UK) displaying diagnostic transitions: TIC (Total ion current); #1 = [M-H]⁻ 706,10>179,20 m/z, CE=29; #2 = [M-H]⁻ 706,20>263.25 m/z, CE=21; #3 = [M-H]⁻ 852,30>161,15 m/z, CE=17. Chart B shows the corresponding MRM profile of LNnT produced by sequential fermentation and retrieved from the culture medium of the last fermentation step. Chart C shows the corresponding MRM profile of LNnT produced by sequential fermentation and retrieved from intracellular fraction of the genetically engineered microbial cells producing the LNnT as desired oligosaccharide.

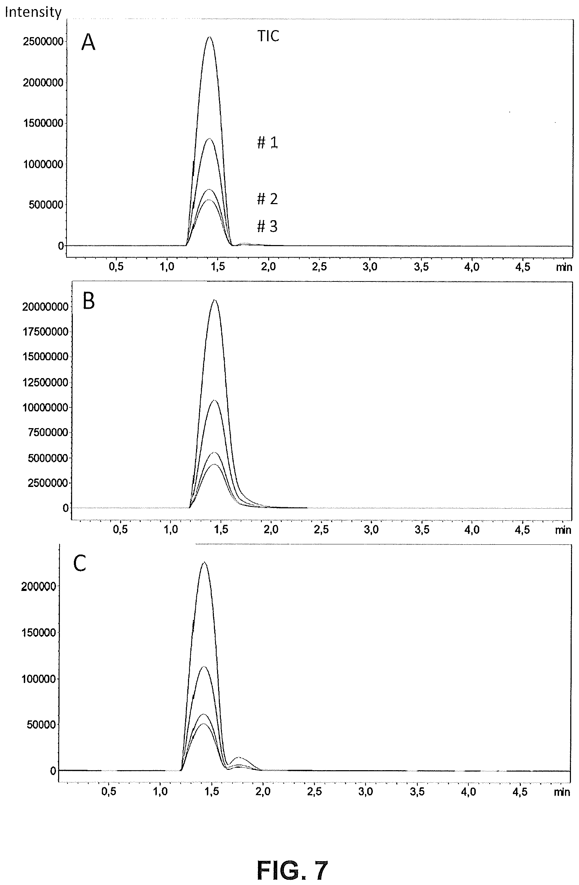

FIG. 7 indicates the identity of LNT-II as confirmed by LCMS based MRM analysis. LNT II was added to a culture of E. coli cells to be converted to LNnT. Chart A shows the MRM profile of commercially available LNT-II standard (Elicityl SA, Crolles, France) displaying diagnostic transitions: TIC (Total ion current); #1 = [M-H]⁻ 544,20>161,00, CE=16; #2 = [M-H]⁻ 544,20>382,10 m/z, CE=11; #3 = [M-H]⁻ 544,20>112,90 m/z, CE=28. Chart B shows the LNT II obtained from the supernatant of the culture medium. Chart C shows the intracellular LNT II obtained from the genetically engineered microbial cell to produce LNT II as an intermediate oligosaccharide.

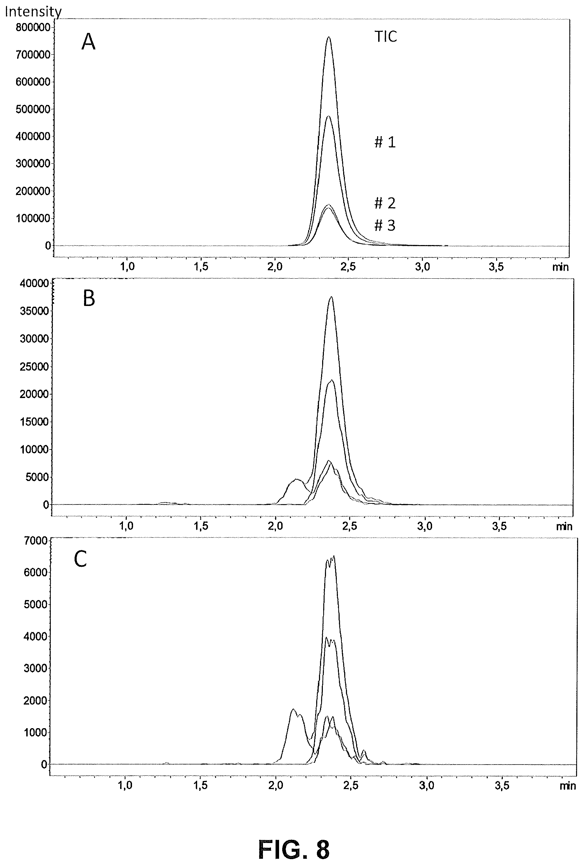

FIG. 8 indicates the identity of LNFP-II produced in a sequential fermentation process described herein by LCMS based MRM analysis. Chart A shows the MRM profile of commercially available LNFP-II standard (Dextra Laboratories Ltd., Reading, PA, USA) displaying diagnostic transitions: TIC (Total ion current); #1 = [M-H]⁻ 852,30>348,20, CE=23; #2 = [M-H]⁻ 852,30>163,05m/z, CE=40; #3 = [M-H]⁻ 852,30>288,20 m/z, CE=29. Chart B shows the corresponding MRM profile of LNFP-II produced by sequential fermentation and retrieved from the culture medium of the last fermentation step. Chart C shows the corresponding MRM profile of LNFP-II produced by the sequential fermentation and retrieved from intracellular fraction of the genetically engineered microbial cells producing the LNFP-II as desired oligosaccharide.

### Example 2: Culture medium used to grow genetically modified E. coli cells:

The culture medium used to grow the cells for the production of the desired oligosaccharides contained: 3 g·L⁻¹ KH₂PO₄, 12 g·L⁻¹ K₂HPO₄, 5 g·L⁻¹ (NH₄)₂SO₄, 0.3 g·L⁻¹ citric acid, 0.1 g·L⁻¹ NaCl, 2 g·L⁻¹ MgSO₄ × 7·H₂O and 0.015 g·L-¹ CaCl₂ × 6·H₂O, supplemented with 1 mL·L⁻¹ trace element solution (54.4 g·L⁻¹ ammonium ferric citrate, 9.8 g·L⁻¹ MnCl₂ × 4·H₂O, 1.6 g·L⁻¹ CoCl₂ × 6·H₂O, 1 g·L⁻¹ CuCl₂ × 2·H₂O, 1.9 g·L⁻¹ H₃BO₃, 9 g·L⁻¹ ZnSO₄ × 7·H₂O, 1.1 g·L⁻¹ Na₂MoO₄ × 2·H₂O, 1.5 g·L⁻¹ Na₂SeO₃, 1.5 g·L⁻¹ NiSO₄ × 6·H₂O). The pH of the medium is 7.0.

### Example 3: Genetically modified E. coli cells to produce oligosaccharides

*E. coli* BL21 (DE3) was engineered for the biosynthesis of fucosylated oligosaccharides by deleting genes encoding enzymes that degrade the acceptor substrates, the donor substrate or interfere with the synthesis of nucleotide activated sugars use in glycosyltransferase reactions, therefor the *lacZ* gene, encoding the β-galactosidase, genes *fucI* and *fucK,* encoding a fucose isomerase and the fuculose kinase, respectively, and the gene *wcaJ,* encoding the first enzyme in the colonic acid biosynthesis pathway in *E. coli* were deleted. For enhanced *de novo* synthesis of GDP-fucose in *E. coli* the genes *manB, manC, gmd,* and *wcaG* encoding the phosphomannomutase, mannose-1-phosphate guanyltransferase, GDP-mannose 4,6-dehydratase, and GDP-L-fucose synthase were overexpressed in the cells. To confer to the strain the capability to synthesize GDP-fucose using the salvage pathway, the gene *fkp* from *Bacteroides fragilis,* encoding the bifunctional fucokinase/guanosine-5-phosphate pyrophosphorylase was functionally integrated into the genome of the *E. coli* strain (*E. coli* Strain I).

For the synthesis of lacto-N-neotetraose in *E. coli* BL21 (DE3) *ΔlacZ* the genes the genes *pgm,* encoding the phosphoglucomutase,as well as *galU* and *galE* coding for the UTP glucose-1-phosphate uridyltransferase and the UDP-glucose-4-epimerase favoring the synthesis of UDP-galactose were overexpressed. To catalyze the transfer of galactose from UDP-galactose onto the intermediate oligosaccharide LNT II to obtain lacto-N-neotetraose the β-1,4-galactosyltransferase *lex-1* from *Aggregatibacter aphrophilus* was constitutively expressed in the *E. coli* strain (*E. coli* Strain II).

### Example 4: Synthesis of LNFP II and LNFP III by transglycosylation

Above mentioned *E. coli* BL21(DE3) derivative Strain I was engineered by genomic integration of the functional gene encoding the loop engineered alpha-1,3/4-L-fucosidase AfcB (Zeuner et al., 2018) from *Bifidobacterium longum subsp. infantis.* The fucosidase gene was constitutively expressed from the promoter Ptet. This strain was grown in mineral salts medium containing 2% glycerol as sole source of carbon and energy at 30°C in baffled shaking flasks for 24 hours. A main culture containing the same medium with 2% glycerol as carbon source and as well 28 mM 3-fucosyllactose and 28 mM lacto-N-tetraose or 28 mM lacto-N-neotetraose was inoculated from the pre-culture to an optical density of 0.2. The culture was incubated under aerobic conditions at 30°C. After 48 h of incubation samples of the cultures were taken, cells from were sedimented by centrifugation, washed once with cold NaCl (0,9% (w/v)), resuspended in ice-cold 50% (v/v) methanol and frozen at -20°C. The supernatant of the cell culture was subjected to LC/MS-analytics to identify the reaction products LNFP II and LNFP III secreted into the medium. Frozen cells were thawed, centrifuged again to sediment the cell debris and the intracellular LNFP II and LNFP III produced was analyzed by LC/MS.

LNFP II and LNFP III was detected in the respective cultures as well in the supernatant as in the cytoplasmic fraction of the *E. coli* cells. The pentaoses were identified by comparing the pattern of fractionations compared to commercial standard substances.

### Example 5: Production of LNFP III from LNnT and L-fucose using a fucosyltransferase reaction

*E. coli* BL21 (DE3) Strain I was used to engineer a cell to produce LNFP III using an appropriate fucosyltransferase. The gene encoding the alpha-1,3/4-fucosyltransferase from *Helicobacter pylori* (Yu, H. et al. (2017). H. pylori α1-3/4-fucosyltransferase (Hp3/4FT)-catalyzed one-pot multienzyme (OPME) synthesis of Lewis antigens and human milk fucosides. Chemical Communications, 53(80), 11012-11015.) was integrated into the genome of the *E. coli host* and constitutively expressed from the transcriptional promoter Ptet.

This strain was grown in mineral salts medium containing 2% glycerol as carbon source for about 24 h at 30°C. Fresh medium containing the same carbon source and as well 2% LNnT as 10 mM L-fucose was inoculated from the pre-cultures to an optical density of 0.04 and incubated for 48h at 30°C under aerobic conditions.

After 48 h of incubation samples of the cultures were taken, cells were sedimented by centrifugation, washed once with cold NaCl (0,9% (w/v)), resuspended in ice-cold 50% (v/v) methanol and frozen at -20°C. The supernatant from the cell culture was subjected to LC/MS-analytics for identification of the reaction product LNFP III secreted into the medium. Frozen cells were thawed, centrifuged again to sediment the cell debris and the intracellular LNFP III was analyzed by LC/MS.

### Example 6: Identification of effective LNT II importers for LNnT production from the metagenomic DNA from human stool samples

Genomic DNA from stool samples from breast-fed infants was isolated using (Quick-DNA Fecal/Soil Microbe Kits, Zymogen, Freiburg, Germany) according to the manufactures protocol. The genomic DNA was shared optionally to fragments of about 40 kbp and end-repaired according to the protocol for the CopyControl™ Fosmid Library Production Kit with pCC1FOS™ Vector (Lucigen, Epicentre, Madison, USA). Transduction was also performed according to the Lucigen protocol in the *E. coli* BL21(DE3) derivative Strain II described above. A transduction efficiency/phage titer of 1x10⁴ cfu's was achieved. Cells were plated onto 2YT medium (Sambrook and Russel, Molecular Cloning: A Laboratory Manual 3rd ed., Cold Spring Harbor Laboratory Press (Cold Spring Harbor, N.Y. 2001) containing 12,5 µg/ml chloramphenicol to select for propagation of the fosmid vector. Colonies were transferred into 96-well plates containing 200 µl mineral salts medium with 2% glucose as carbon source and 12.5 µg/ml chloramphenicol for antibiotic selection. After 24 h of growth at 30°C 50 µl of the culture were transferred into fresh medium containing 2% glucose, 20 mM lacto-N-triose II (98 % purity as determined by HPLC) 0.1 mM IPTG (isopropyl-p-thiogalactopyranoside) and 12.5 µg/ml chloramphenicol. After about 30 h of growth at 30°C cells were harvested by centrifugation, the supernatant was heated for 5 min at 95°C and insoluble particles were sedimented by centrifugation. The cleared supernatant was subjected to LC/MS analysis for the detection of LNnT production. About 0.5 % of the clones screened for LNnT production showed a 3-fold to 5-fold enhanced LNnT production compared to base line.

Fosmid DNA of clones showing LNnT production were isolated from the cells, retransformed into the same strain and tested a second time. If enhanced LNnT production was verified, the plasmid DNA was sequenced, the gene of interest subcloned and functionality was proofed again by measuring LNT II internalization and LNnT production.

## Claims

1. A process for the production of a desired oligosaccharide comprising more than three monosaccharide moieties, wherein the process comprises
- providing a genetically engineered microbial cell that possesses
∘ a saccharide importer for the uptake of an intermediate oligosaccharide consisting of at least three monosaccharide moieties but less monosaccharide moieties than the desired oligosaccharide by the microbial cell, and
∘ an enzyme which is able to transfer a monosaccharide moiety from a donor substrate to the intermediate oligosaccharide;
- cultivating the genetically engineered microbial cell in a culture medium which contains the intermediate oligosaccharide for the genetically engineered microbial cell to take up the intermediate oligosaccharide and to transfer at least one monosaccharide moiety to the intermediate oligosaccharide, thereby synthesizing the desired oligosaccharide; and
- retrieving the desired oligosaccharide from the culture medium and/or the genetically engineered microbial cell.

2. The process according to claim 1, wherein the enzyme which is able to transfer a monosaccharide moiety from a donor substrate to the intermediate oligosaccharide is glycosyltransferase or a transglycosidase.

3. The process according to claim 1 or 2, wherein the donor substrate is a nucleotide-activated sugar or an oligosaccharide.

4. The process according to claim 3, wherein the nucleotide-activated sugar is selected from the group consisting of GDP-fucose, UDP-galactose, UDP-glucose, UDP-N-acetylglucosamine, UDP-N-acetylgalactosamine, CMP-N-acetylneuraminic acid.

5. The process according to any one of claims 1 to 4, wherein the donor substrate is synthesized by the genetically engineered microbial cell and/or is exogenously added to the culture medium.

6. The process according to any one of claims 1 to 5, wherein the intermediate oligosaccharide is exogenously added to the culture medium.

7. The process according to any one of claims 1 to 6, wherein the intermediate oligosaccharide has been produced by microbial fermentation.

8. The process according to any one of claims 1 to 7, wherein the genetically engineered microbial cell lacks enzymatic activity liable to degrade the intermediate oligosaccharide.

9. The process according to any one of claims 2 to 8, wherein the glycosyltransferase is selected from the group consisting of fucosyltransferases, galactosyltransferases, glucosaminyl transferases, sialic acid transferases, N-acetylglucosaminyl transferases, N-acetylglucosaminyl transferases.

10. The process according to any one of claims 2 to 8, wherein the transglycosidase is selected from the group consisting of alpha-1,2-fucosidase, alpha-1,3/1,4-fucosidase, alpha-1,4 glucosidase, alpha-1,6 glucosidase, alpha-1,3 glucosidase, beta-glucosidase, and variants of a transglycosidase lacking hydrolase activity.

11. The process according to any one of claims 1 to 10, wherein the intermediate oligosaccharide is LNT-II, and the desired oligosaccharide is a tetrasaccharide selected from the group consisting of LNT and LNnT.

12. The process according to any one of claims 1 to 11, wherein the intermediate oligosaccharide is LNnT and the desired oligosaccharide is LNFP-III, or wherein the intermediate oligosaccharide is LNT and the desired oligosaccharide is LNFP-II.

13. The process according to any one of claims 1 to 12, wherein the genetically engineered microbial cell is a yeast cell, preferably a yeast cell selected from the genera *Saccharomyces, Schizosaccharomyces, Pichia, Hansenula,* or a prokaryotic cell, preferably a bacterial cell selected from the group consisting of *Escherichia sp., Bacillus sp.* and *Campylobacter sp.*

14. The process according to any one of claims 1 to 13, wherein the donor substrate is a nucleotide-activated sugar, preferably a nucleotide activated sugar selected from the group consisting of GDP-fucose, UDP-galactose, UDP-glucose, UDP-N-acetylglucosamine, UDP-N-acetylgalactosamine and CMP-N-acetylneuraminic acid, or an oligosaccharide.

15. A process for the production of lacto-N-neotetraose, wherein the process comprises
- providing a genetically engineered microbial cell that possesses
a. a transporter which facilitates uptake of LNT-II, and
b. a β-1,4 galactosyltransferase;
- cultivating the genetically engineered microbial cell in a culture medium which contains LNT-II for the genetically engineered microbial cell to take up LNT-II and to transfer a galactose moiety from UDP-galactose to LNT-II by said β-1,4 galactosyltransferase, thereby synthesizing LNnT; and
- retrieving the LNnT from the culture medium and/or the genetically engineered microbial cell.
